Europäisches Patentamt

European Patent Office

Office européen des brevets

(19)

(11)  EP 0 402 877 B1

(12)  **EUROPEAN PATENT SPECIFICATION**

(45) Date of publication and mention
of the grant of the patent:
**04.09.1996  Bulletin 1996/36**

(51) Int Cl.$^6$: **G01N 33/12**

(21) Application number: **90111132.8**

(22) Date of filing: **12.06.1990**

(54) **Method and apparatus for photometric determination of properties of meat pieces**

Verfahren und Vorrichtung zur photometrischen Bestimmung von Eigenschaften von Fleischstücken

Méthode et appareil pour la détermination photométrique de caractéristiques de morceaux de viande

(84) Designated Contracting States:
**AT BE CH DE DK ES FR GB GR IT LI LU NL SE**

(30) Priority: **12.06.1989  DK 2852/89**

(43) Date of publication of application:
**19.12.1990  Bulletin 1990/51**

(73) Proprietor: **Slagteriernes Forskningsinstitut
DK-4000 Roskilde (DK)**

(72) Inventors:
• **Andersen, Jan Rud
  DK-4060 Kirke Saaby (DK)**
• **Borggaard, Claus
  DK-4130 Viby Sjaelland (DK)**

(74) Representative: **Beetz & Partner Patentanwälte
Steinsdorfstrasse 10
80538 München (DE)**

(56) References cited:
**EP-A- 0 221 642          EP-A- 0 286 142
WO-A-80/01205**

• **VOEDINGSMIDDELEN TECHNOLOGIE, vol. 17,
no. 24, November 1984, pages 37-38, Zeist, NL;
"Infrarood analyse bij de kwaliteitsbewaking van
vleeswaren"**
• **IDEM**

EP 0 402 877 B1

**Description**

The present invention relates to a method and an apparatus for the photometric determination of properties of individual pieces of meat wherein the optical properties of the meat are measured in a wide wavelength range, and the measured values are evaluated by means of a microcomputer system.

The colour of meat is often considered to be a quality parameter. For some types of meat the consumers prefer light-coloured meat, and for other types of meat they wish a strong colour. On certain markets the customers demand that pigmeat delivered must be strongly coloured as this ensures that the buyer is not supplied with the so-called PSE-meat, which has a light colour and a poor juice-binding capacity, due to a low pH-value shortly after slaughtering. Normally, the colour does not affect the quality of pigmeat, since normal light-coloured meat has the same juice-binding capacity as dark meat. For the above reasons, difficulties may arise on demanding markets, however, as to selling the light-coloured but otherwise normal pigmeat, which may be mistaken for PSE-meat.

For slaughterhouses and the meat processing industry it may be essential to prove to the customers that the light meat colour is due to a low content of pigment in the meat, and that the meat quality is in order. In the manufacture of processed meat products it is also very important to indicate that the light meat colour is caused by a low content of pigment, so that the meat may be used in the normal production. It would be too expensive having to sort out all light-coloured pieces of meat for an inferior production. Myoglobin and residual haemoglobin constitute the pigment of the meat.

There are known methods of analysis for determination of the content of pigment in pigmeat, but these methods are so laborious and time-consuming that they are inapplicable for control under production conditions.

Also photometric methods for determination of the colour of meat are known, but these are not suitable to establish whether a light colour of pigmeat is due to a low content of pigment.

Danish Patent Application No. 4211/86 describes an instrument for the classification of fish meat according to colour.

The instrument comprises a source of light for the illumination of the fish meat, a prism system to disperse the reflected light into a spectrum, and a line of photodiodes which are placed in the spectrum in such a way that each diode receives light at its own individual wavelength. The intensity of the light in the spectrum is registered at the wavelengths where haemoglobin has a special light absorption characteristic, the diodes corresponding to these wavelengths being connected with a reading and memory unit. The light intensity is registered at 540-580, 425 and 625 nm. The latter is used as a reference.

The registered values are compared by means of a microprocessor to the values of three stored sets of values, each representing a class of colour. The class of colour of the fish meat is determined as the set which shows the best agreement between the registered values and the stored values.

Another device and test method for the determination of the colour of pigmeat and veal meat are described by H.J. Swatland in Can. Inst. Food Sci. Technol. J. 19, (1986), pages 170-173. The instrument is provided with a probe which is designed to be inserted into the piece of meat, the colour of which is to be measured. In the probe there are two bunches of optical fibres which produce the illumination of the meat and which lead the reflected light into a prism system.

In one of the tests described the reflection of pigmeat is measured in the range of 400-700 nm. The pigmeat used was selected as a representative of meat with an ideal colour and meat with some degree of PSE. As was to be expected, it is ascertained that the PSE-meat is lighter in colour than the ideally coloured meat over the whole range of wavelengths. The article does not deal with the content of pigment in pigmeat and the risk of mistaking meat with a low content of pigment for PSE-meat. Accordingly, this method applied is not suitable for detecting whether the light colour of pigmeat is due to a low content of pigment.

A method and an apparatus for the photometric determination of properties of individual pieces of meat, particularly the determination of the color grade of fish flesh is known from EP-A3-221 642.

This prior art method is based on the determination of hemoglobin which is the dominant colorant in fish fillets. The light reflected from the sample is measured at a plurality of frequencies at which hemoglobin displays light absorption characteristics. This set of measured intensity values of reflected light is compared with preestablished sets of these values, and the color grade of the sample is determined by determining the closest stored set of values.

It is the object of the present invention to provide a quick method and an apparatus for the determination of properties of meat, including fish meat, and particularly the content or concentration of one or more substances, such as the pigment, in individual pieces of meat, particularly pigmeat.

On-line application of the method on a slaughterline or production line should be possible, ensuring a quick result of determination so that the pieces of meat may be graded or marked immediately in accordance with the determined property, particularly the content of pigment.

The above object is achieved according to the independent claims. The dependent claims relate to preferred embodiments.

The property C of meat to be determined is preferably the concentration of a substance comprised in the meat, which is calculated from the measured intensities of reflected light $R_1$ to $R_n$.

The measuring means preferably comprise dispersion means for dispersing the reflected light in dependence of the wavelength, preferably a spectrophotometer.

The method of the present invention is based on the registration of a large number of values of reflection over a wide range of wavelengths. In addition, all the values are used and inserted into a suitable equation or equation system, which is preferably a multi-variable calibration model or algorithm, for the calculation of the respective property. The advantage of this method is that it is possible to eliminate to a high degree disturbing signals from other substances than the one or those being measured, since the model or equation or equation system (in the following simply referred to as algorithm) is based upon and utilizes the values of reflection at all the wavelengths of the selected range. They all contribute information increments relating to one or more substances. This is quite different from the photometric methods usually being applied, where the noise is eliminated by narrowing the width of the range of measurement.

It has been shown that the method according to the invention is suited for determining the content of different substances in meat, e.g. the content of pigment. The pigment content is determined with a higher accuracy than by conventional photometric methods which are based upon the value of reflection at one wavelength or upon one value of reflection which covers a very wide range of wavelengths.

In order to ensure a high accuracy, registration of the optical values should be performed in the entire, wide range of wavelengths which by estimation or tests has been determined to be of interest, and all the registered values should be used and inserted into the algorithm which should have a corresponding number of variables. The wavelengths $\lambda_1$ to $\lambda_n$ are preferably approximately evenly distributed over the respective wavelength range, thus covering a continuous wavelength range.

The fundamental rule of the method of the invention is that the value of reflection intensity (or the reflectance, which is the normalized reflection intensity) should be determined for all the wavelengths of the selected range, and that the algorithm should also have its full size with many variables. Only if it can be proved positively that some bands have a negative effect or are without any effect, they may be omitted. For reasons relating to the complexity of the instrument or the calculation some wavelengths may be omitted, e.g. every other wavelength or wavelengths of a band which are situated on the fringe of the selected range.

There are mathematical models and software which make it possible to develop a multi-variable algorithm which is suitable for the calculation of the concentration of a substance. They are known under the designation of multi-variable calibration models, see Example 1, part D (Algorithms).

The meat surface may be the outer, cut surface of a meat piece, or the surface resulting from inserting a needle- or rod-shaped probe having an optical window.

The method according to the invention may be used to determine in meat the concentration of a substance that has an absorption curve which is different from that of other substances of the meat. The determination could for instance concern the concentration of pigment, protein, fat or water. The concentrations of these substances form essential parameters of meat quality.

The present method may be applied to meat, such as red meat from pigs, bulls, young steers, calves, deer, sheep, lambs or other tissue parts of slaughtered animals.

In a preferred embodiment of the invention the number of wavelengths ($\lambda_1$ to $\lambda_n$) and variables is 15 or more and preferably 30 or more. The application of a low number of variables will simplify the procedure of calculation, but the accuracy of the determination will be decreased. It has been shown that a good accuracy of determination may still be achieved by using only 32 variables.

The number of wavelengths and variables used may be lower than 600, and preferably lower than 300. The application of a high number of variables may involve the risk of an overinterpretation of the values of reflection, but on the other hand, the accuracy of determination is increased by using a correct, complete algorithm. It has been shown that the calculation may be made within the time available, and that a very good accuracy of determination may be obtained by applying 128 variables.

The accuracy of the method may also be improved by registration of the values of reflection intensity in a wide range of wavelengths, so that a substantial part of the absorption curves for the substances of interest comprised in the meat falls within that range. Preferably, the series of different wavelengths comprises a range of at least 100 nm and particularly of at least 200 nm.

A substantial part of the absorption curve of the substances often falls within the visible range, as is the case with for instance the determination of pigment. In a preferred embodiment of the present invention, the series of wavelengths comprises a part, e.g. the main part, of the visible light region. This covers the range from 250 to 800 nm.

In the near-infra-red region (NIR) certain substances have characteristic curves of absorption, e.g. proteins, fat and water. The series of different wavelengths may therefore comprise wavelengths in the NIR region, covering the range from approx. 800 to approx. 2500 nm.

In this specification, by the term wavelength it is to be understood not exclusively a discrete wavelength but also a number of wavelengths comprising a narrow or discrete band. The width of each of these bands is preferably 1 to 20 nm. This gives sufficiently detailed information of the course of the curve of reflection. The bands need not necessarily be of the same width throughout the spectrum or the continuous wavelength range.

For optical reasons the width of the bands at short wavelength will often be smaller than that of bands at the long-wave end of the region. If desired, the values of reflection intensity may be processed (e.g. normalized) so that the differences are smoothened.

In accordance with a preferred embodiment the multi-variable model or algorithm corresponds to the following formula:

$$C = k_0 + k_1 \cdot R_1 + k_2 \cdot R_2 + k_3 \cdot R_3 \dots k_n \cdot R_n$$

wherein C is the property of interest, particularly the concentration of a substance of the meat, $R_1$ to $R_n$ are the intensities of reflected light measured at the respective wavelengths ($\lambda_1$ to $\lambda_n$), and $k_0$ to $k_n$ are predetermined constants.

The multi-variable equation or algorithm is preferably provided by multi-variable calibration model by means of a set of calibration data consisting of values of optical properties measured at a plurality of different wavelengths and reference values of the respective properties, e.g. the concentrations of the substance. The values of the reflection intensity at the different wavelengths are determined on a number of samples, e.g. 50 samples of meat. Preferably, there is a good span in the sample material. The concentration of the substance of interest is then determined, such as the content of pigment, in each of the samples by a reliable, well-known method of analysis. The data obtained may easily be processed in a computer program. The program will calculate the constants of the multi-variable algorithm as those values which in toto give the best agreement between the values of the concentrations calculated by means of the algorithm and the reference values.

Reference values for another substance of the samples may also be fed into the program, such as the content of fat, and then the program calculates the constants of another model or algorithm. This algorithm may then be used to determine the concentration of this other substance in the meat.

A special mathematical method that is applied in connection with multi-variable calibration is called the partial least squares method (PLS method). This method makes it possible to detect, among other information, whether a sample or a determination is outside what is known from the samples that were used for the calibration. In accordance with this, the algorithm may be determined by the partial least squares method (PLS).

The apparatus of the present invention comprises an instrument for the measurement of the optical properties of the meat, and a computer unit designed to receive the measured values and for inserting them into a mathematical equation or model (algorithm) and performing the respective calculation.

The apparatus according to the invention is preferably designed to perform the reflection intensity measurements over a continuous wavelength range. The algorithm stored in the computer unit is preferably a multi-variable algorithm expressing e.g. the concentration of a substance of the meat, and the optical reflection intensity values measured are automatically inserted as variables into the mathematical model.

The method and the apparatus of the present invention make it possible to determine for instance the concentration of pigment in individual pieces of pigmeat very quickly and accurately. Thus, it will be possible to show whether the light colour of a piece of meat is due to a low content of pigment, which has not been possible with methods and apparatus known so far. The apparatus may perform the determination on-line on a slaughter or production line, and the result will be at hand so quickly that the meat may be graded or marked immediately in accordance with the concentration of the pigment found.

In an embodiment, the computer unit includes a program which may provide or adjust the algorithm by multi-variable calibration. The optical values which are used in the calibration may come from the apparatus itself or from other similar apparatus.

An apparatus which includes such a program is easy to adjust and to adapt to new purposes. If desired, it may be made self-adjustable.

The computer unit may comprise an output channel which is designed to transmit control signals controlling the transport route of an examined piece of meat and/or to actuate a marking or printing device for the respective piece of meat.

Consequently, it is possible by means of the apparatus according to the invention to grade or classify meat depending on their different concentration of interesting substances fully or semi-automatically.

In the following, the invention will be described with further details with reference to the drawings, in which

Fig. 1      illustrates an apparatus for the determination of e.g. the content of pigment in individual pieces of meat;

Fig. 2      is a diagram illustrating the correlation between the analytically determined content of pigment and the calculated content of pigment by application of an algorithm with 128 variables;

Fig. 3      is a diagram corresponding to that of Fig. 2, but relating to the application of an algorithm with 32 variables;

Fig. 4      is a diagram, serving as a comparison, of the analytically determined content of pigment in relation to the reflectance of the samples at 552 nm;

Fig. 5      is a diagram showing the influence of the factors which are included in the algorithm with 128 variables;

Fig. 6      is a diagram corresponding to that of Fig. 5 showing the influence of the factors in connection with an algorithm with 32 variables;

Fig. 7     is a spectrum of reflection in the NIR region of 1200-2400 nm;

Fig. 8     is a diagram illustrating the correlation between the analytically determined protein content and the calculated protein content in Longissimus dorsi muscles, and

Fig. 9     is a diagram corresponding to that of Fig. 8, but relating to Biceps femoris muscles.

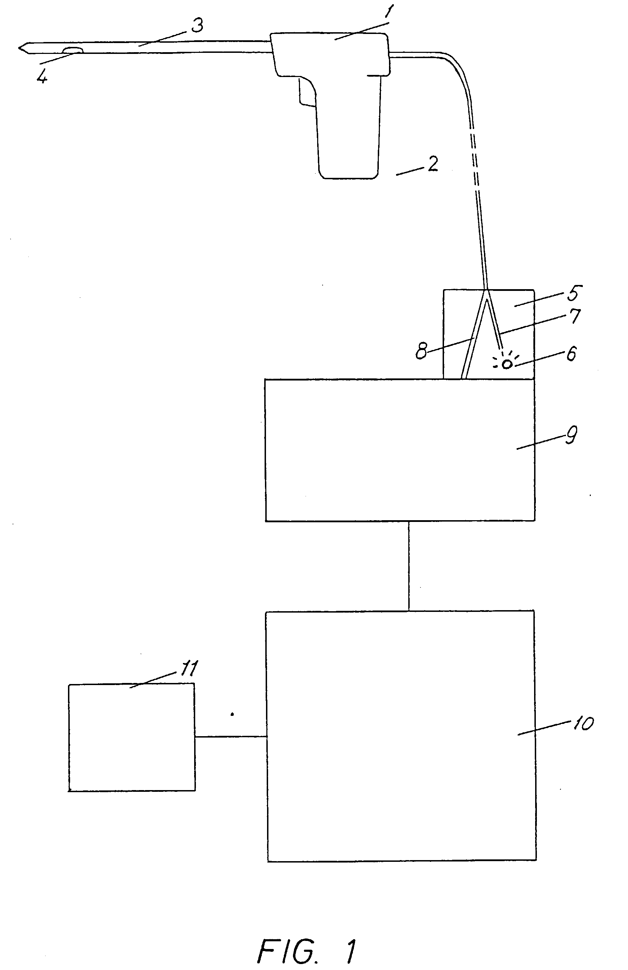

The apparatus shown in Fig. 1 is a prefered embodiment and comprises a pistol-like instrument 1 with a handle 2 and a probe 3 which is inserted into the piece of meat whose content e.g. of pigment is to be measured. The side of the probe is provided with a window 4.

A box 5 is provided with a lamp 6 of xenon or halogen type which via a set of optical fibres 7 may illuminate the meat outside the window 4. Another set of optical fibres 8 leads the light reflected from the meat through the window 4 to a spectrophotometer 9. The spectrophotometer is provided with a grating which disperses the light into different wavelengths. The diffracted light hits a plate with a line of photodiodes which each register the intensity of the light in the corresponding wavelength band.

Alternatively, a single, quickly registering detector may be applied. Further, a scanning spectrophotometer may be used.

The pistol-like instrument 1 may also be provided with a lamp and a detector.

A computer 10 is connected to the spectrophotometer for transfer of data and starting pulses. Via control circuits the computer is also connected to the power supply of the lamp and a button on the instrument 1. A push on the button starts the measuring procedure of the computer and releases a flash of light from the lamp 6, and also ensures that the light reflection signals are transferred from the spectrophotometer 9 to a temporary memory in the computer.

A computer program controls the measuring procedure. The program may include an error routing. Via a display 11 the operator is informed of the steps in operation. It realizes whether a measuring made may be accepted.

After a provisional approval of a measuring, the program ensures that the results of the measurement are transferred to a calculation program in the computer. The optical values obtained for the meat are inserted into an algorithm which has been stored in the program. The program will calculate a value for the quality property being examined, such as the content of pigment, and it will check whether the value found is within the acceptable limits. Via the display 11 the computer will inform the operator of the result, and, if required, transfer it to other computers. The display may e.g. show the operator how big the content of pigment is, expressed in ppm, or whether the piece of meat has a content which is within the preset limits, and optionally, into which group the piece of meat is to be graded.

The computer may comprise software which provides for the registration of e.g. the number of the piece of meat, and which gives information of this as well as of the result.

Via the output channel of the computer it is possible to control automatic marking, printing or grading devices in accordance with the result.

If desired, the computer may perform another calculation cycle with the values by means of another algorithm expressing another property of the meat, e.g. the content of protein. Consequently, the same results of reflection intensity measurements may be used for the determination of one, two, or eventually more quality properties, depending on the demands and the limitation of the measuring method.

The computer may comprise a calibration program which in its most simple form transfers a new or adjusted algorithm to the calculation program.

Another embodiment of the program may be designed to make its own calculation of the algorithm on the basis of a number of calibration measurements made on pieces of meat with a known composition or content of substances of interest. By means of a mathematical or statistical model the program may perform an analysis of the measured values of reflection and the corresponding true values, which are fed into the computer, and to calculate corresponding formula parameters and/or constants.

By means of this calibration method, minor mechanical, optical or electrical errors in the individual apparatus may be eliminated.

In the following, the invention will be described by way of examples with reference to the drawings.

Example 1

Determination of pigment

Cuts of loin from 23 pig carcasses are used for this test. Photometric measurements of the loins are performed by means of the apparatus described above. The probe is inserted into the meat at the hip section and at the middle section. The reflection intensity at the different wavelength bands is registered by 512 photodiodes provided in the spectrophotometer. The total range of wavelengths is 360 to 780 nm. Preliminary examinations have shown that the relevant information lies within the range of 400 to 655 nm.

A - Algorithm with 128 variables

Based on the approved measurements, the computer program reduces the number of registered values per measurement to 128. The program groups the photodiode values four by four, and the average value of the four values of each photodiode set is calculated.

The obtained values are put into an algorithm which expresses the content of pigment by means of the 128 values of light reflection intensity at the different wavelengths. The calculated content of pigment is printed out or shown on the display 11.

Two samples of meat are taken from each loin at the spots of insertion, one at the hip section and one at the middle section. The samples are analysed for the content of pigment according to a variant of a known method (H.C. Hornsey, J.Sc.Food Agric. 7, (1956) 534-540). This modified method ensures that the measurement is performed on constant dilution, solvent being supplemented after the filtration.

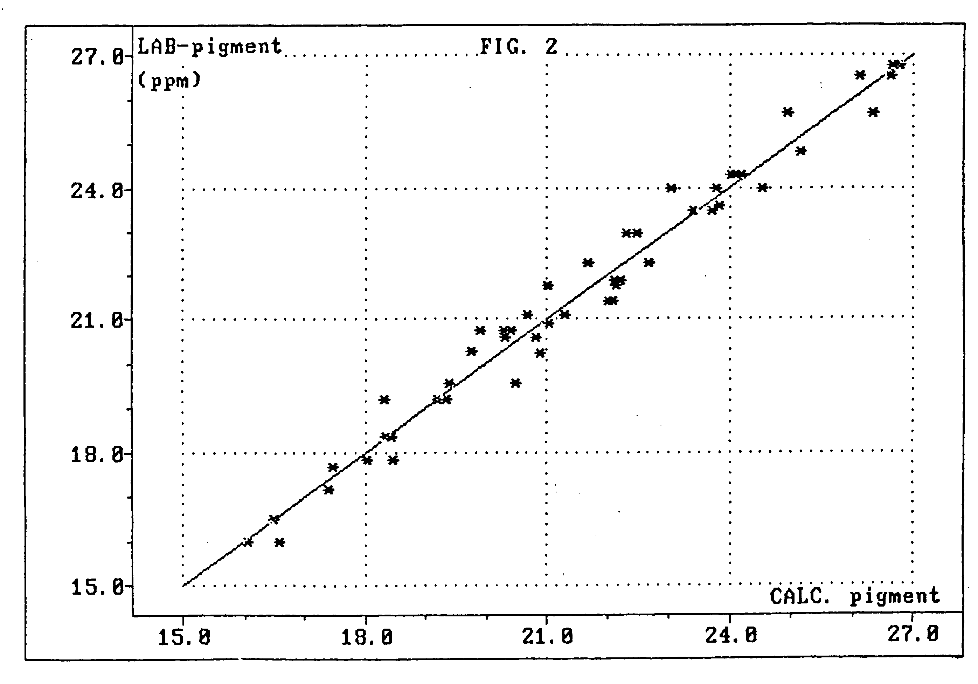

The analytically determined content of pigment of the loins and their calculated content of pigment are illustrated in the diagram of Fig. 2. The points represent 50 probe measurements.

As may be seen, there is a very high correlation between the analytically determined values ('lab pigment') and the calculated values ('calc. pigment').

### B - Algorithm with 32 variables

Based on the same measurements of reflection as described above the computer program reduces the number of values per measurement to 32, the photodiode values being grouped 16 by 16, and the average is calculated. The values obtained are put into an algorithm with 32 variables, and the calculated content of pigment is plotted into a diagram together with the analytically determined content of pigment.

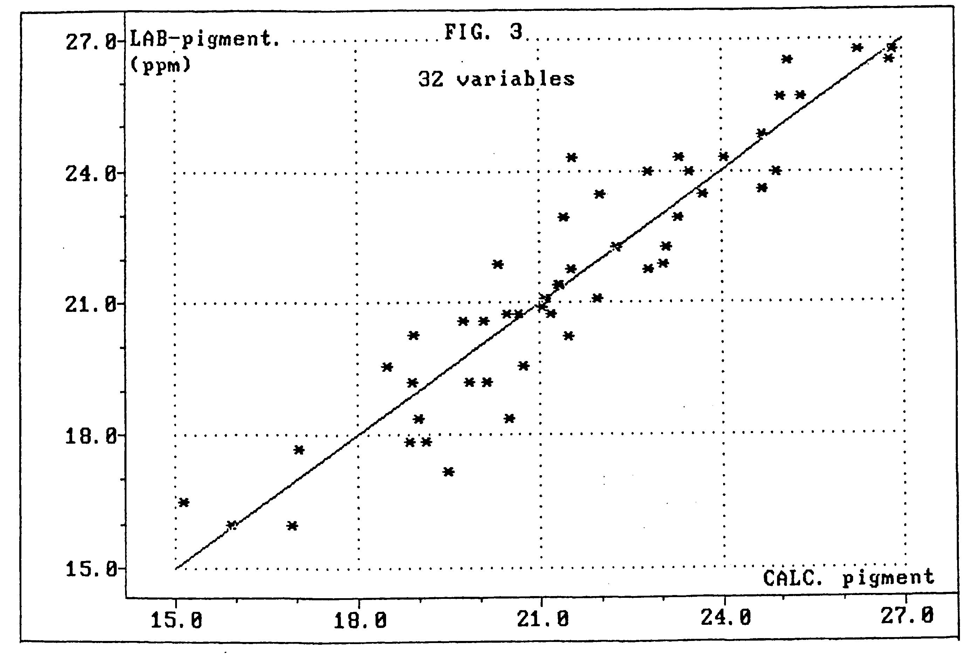

As will appear from Fig. 3, a high correlation is also achieved with the reduced number of variables.

### C - Comparison

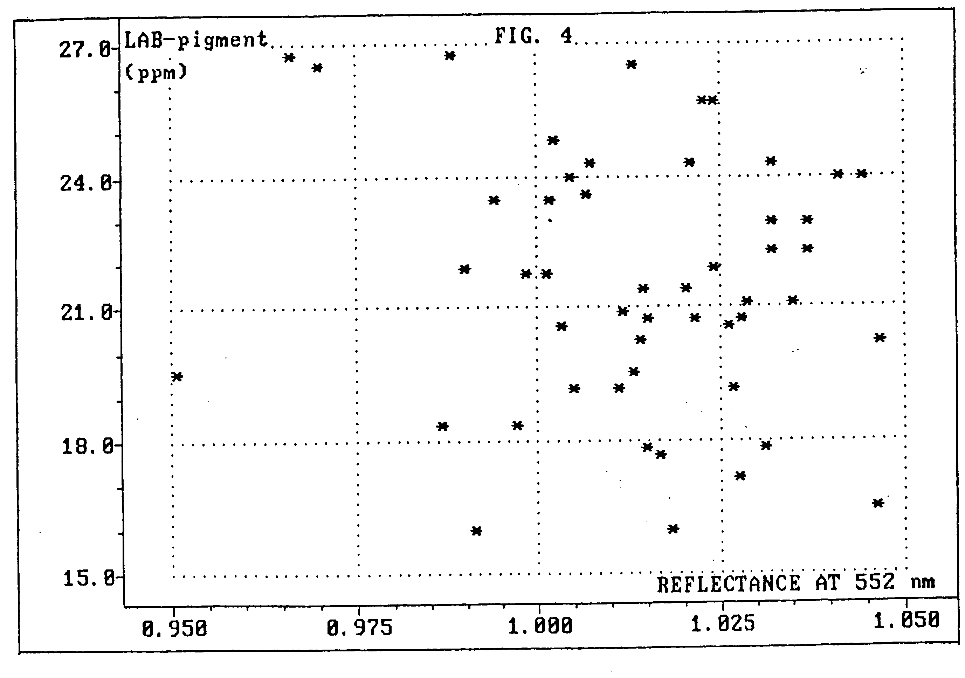

By way of comparison it was examined whether there is any connection between the reflectance measured at the wavelength of 550 nm (552 nm), which is characteristic for myoglobin, and the analytically determined content of pigment. The points from the same probe measurements as those above were plotted into Fig. 4. This corresponds to the well-known technique where the algorithm has only one variable.

As may be seen, there is a very poor correlation between the measured value and the analytically determined content of pigment.

### D - Algorithms

For the construction and testing of the equations or algorithms applied above a program called "Unscrambler" is applied, which includes the necessary instructions for the construction of different multi-variable calibration models which are based on the application of the PLS method.

The program makes use of a training or calibration set consisting of the reflection values in n different bands, together with the corresponding analytically determined values for pigment. It calculates the constants of the equation/algorithm which gives the best prediction of this calibration set. Algorithms with a different number of coefficients/constants may be examined. These are expressive of phenomena affecting the variation of the values of reflection. The number of factors giving the best equation is determined by a so-called cross-validation.

The equation/algorithm which is provided by means of the above-mentioned mathematical analysis, and which is applied in above sections A and B, has the following formula:

$$C_{pig} = k_0 + k_1 \cdot R_1 + k_2 \cdot R_2 + k_3 \cdot R_3 + .... k_n \cdot R_n$$

wherein $C_{pig}$ is the content of pigment expressed e.g. in ppm, $k_0$ to $k_n$ are the constants calculated by the program, and $R_1$ to $R_n$ are the values of reflection intensity at the different wavelengths.

The data put into the program may be pre-processed in order to reduce their number, to counteract systematic errors and/or to provide an algorithm with even better predictions. Transformations, such as -logR and multiple scatter correction (MSC) may be used.

When the program has determined the constants of the equation by processing the calibration set, the equation or algorithm may be tested by feeding in new values of reflection. The agreement between the calculated and the analytically determined value of pigment may then be ascertained. The 50 sets of reflection values, for which the content of pigment has been calculated and plotted into the Figures 2 and 3, also served as calibration sets for the provision of algorithms in A and B.

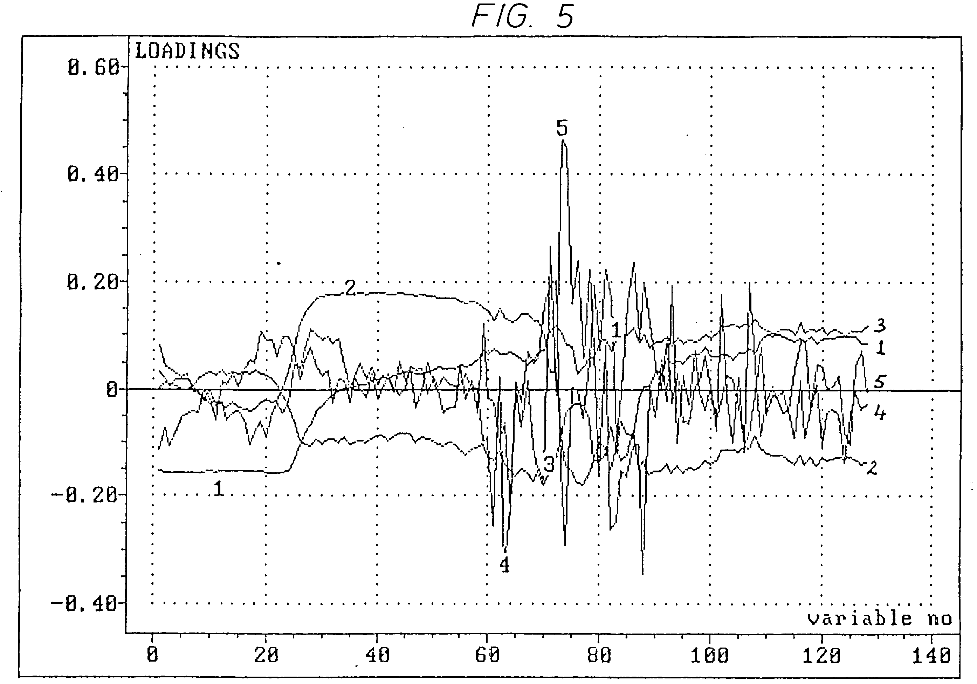

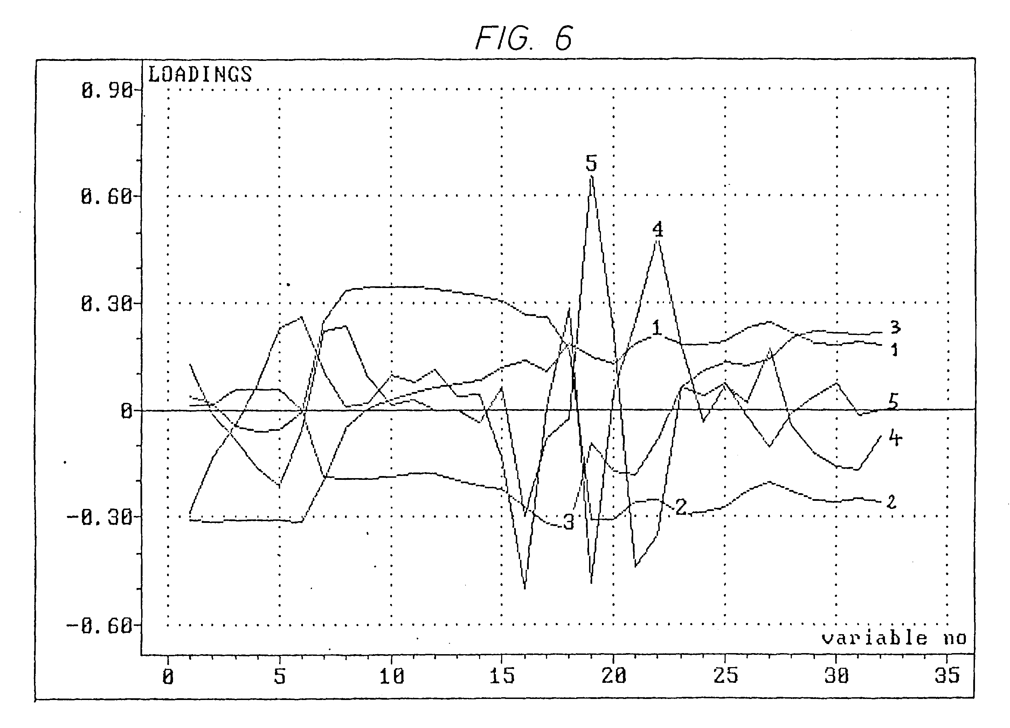

It has been shown that the internal structures of the two algorithms applied are almost identical, which appears from Figures 5 and 6 which illustrate loading curves for the five functions applied. The loading of a function is the weight with which it contributes to the algorithm in the different dimensions. The loading curves of Fig. 5 are more detailed than those of Fig. 6, as 128 variables are applied in the first algorithm, whereas only 32 are applied in the second algorithm.

However, in the main the two loading curves are identical. It is also evident that there is a high loading of the fifth factor in the range of 540 to 570 nm. This wavelength range is the region in the absorption spectrum where absorptions from myoglobin may be expected. This illustrates that both of the algorithms actually "apply" the content of myoglobin in meat.

### Example 2

Determination of protein

49 carcasses are used for this test. Photometric measurements are made in different muscles by means of the same probe and method as in Example 1. The probe is inserted into the muscle whose content of protein is to be determined, and 1,200 reflection intensity values are recorded in the wavelength range of 1,200 to 2,400 nm. The 1,200 values are reduced to 120 values, since every tenth wavelength is selected.

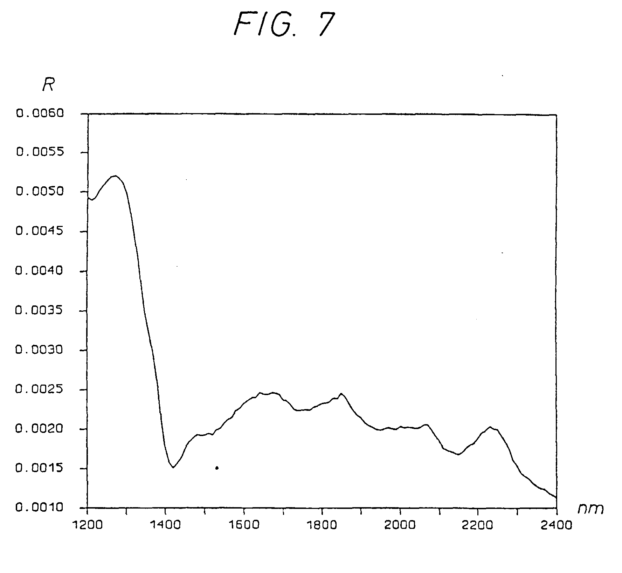

Fig. 7 illustrates a typical spectrum of reflection intensities R obtained in this way from a meat surface. As may be seen, the absorption curve has no distinct peaks. There is no narrow wavelength range to be seen, in which there might be a linear correlation between the absorption of light and the content of protein.

Longissimus Dorsi Muscles

Measurements of reflections are recorded in 19 different muscles. The content of protein of each muscle is determined by Kjeldahl analysis. By means of a PLS-analysis of all data sets of the reflection intensity values and the corresponding Kjeldahl values, an equation/algorithm is determined for the content of protein (model 1). Another equation/algorithm is provided in the same way for the content of protein (model 2). However, in the latter the rough data registered are processed with -logR and multiple scatter correction before PLS-analysis.

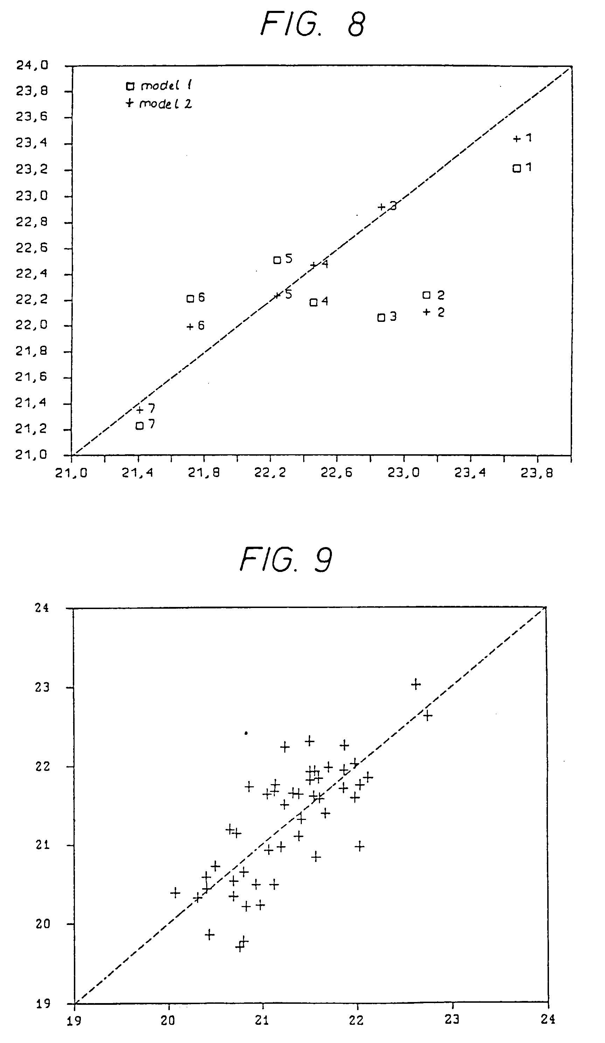

The reflection values for seven muscles chosen at random are put into the equations (models 1 and 2). The results are illustrated in the diagram of Fig. 8, where the Y-axis represents the calculated protein content, and the X-axis represents the analytically determined protein content. As will appear from the Figure, there is a good correlation between the optically and the chemically determined values for the content of protein. The correlation coefficients are 0.84 for model 1, and 0.88 for model 2.

Biceps Femoris Muscles

Measurements of reflections are recorded in 40 different biceps femoris muscles. The content of protein in each of the muscles is determined by means of Kjeldahl analysis. A multi-variable algorithm is formed in the same way as described above.

The results of the measurements of reflections are then put into the algorithm, and the calculated values are compared with the analytically determined values. Fig. 9 illustrates that also for this muscle there is a good correlation between the optically and the analytically determined protein values. The correlation coefficient is 0.76.

**Claims**

1. A method for the photometric determination of the concentrations of one or more substances in a piece of meat comprising:

   - illuminating a meat surface,
   - measuring the intensity of light reflected from the meat surface at a plurality of different wavelengths ($\lambda_1$ to $\lambda_n$) within a predetermined wavelength range, and
   - calculating the concentration of a substance on the basis of the measured intensities of reflected light ($R_1$ to $R_n$) in accordance with a multi-variable algorithm,

   characterized in that

   - a probe is inserted in the piece of meat whose content is to be measured, said probe being provided with a window, a first set of optical fibers illuminating the meat outside said window, and a second set of optical fibers leading the light reflected from the surface of the meat through the window to a spectrophotometer registering the light intensities at different wavelengths,
   - the intensities of reflected light ($R_1$ to $R_n$) are measured at different wavelengths on a meat surface resulting from the insertion of the probe in the meat,
   - the registered intensities are transferred to a calculating program in a computer and inserted in a multi-variable algorithm expressing the concentration of the substance, and
   - the concentration is calculated.

2. The method according to claim 1, characterized in that the concentration is calculated in step II in accordance with the following equation:

$$C = k_o + k_1 \cdot R_1 + k_2 \cdot R_2 + k_3 \cdot R_3 + .... k_n \cdot R_n$$

   wherein

   - $R_1$, $R_2$, $R_3$, .... $R_n$ are the intensities of reflected light measured at the respective wavelengths $\lambda_1$, $\lambda_2$, $\lambda_3$, .... $\lambda_n$, and
   - $k_o$, $k_1$, $k_2$, $k_3$, .... $k_n$ are predetermined constants.

3. The method according to one of claims 1 to 2, characterized in that the intensities of reflected light ($R_1$ to $R_n$) are measured at 30 or more wavelengths ($\lambda_1$ to $\lambda_n$).

4. The method according to one of claims 1 to 3, characterized in that the intensities of reflected light ($R_1$ to $R_n$) are measured at wavelengths ($\lambda_1$ to $\lambda_n$) within a range of at least 100 nm and preferably at least 200 nm.

5. The method according to one of claims 1 to 4, characterized in that the intensities of reflected light ($R_1$ to $R_n$) are measured at different wavelengths ($\lambda_1$ to $\lambda_n$) which comprise a continuous wavelength range, and preferably being approximately evenly distributed over the predetermined wavelength range, preferably each of the wavelengths ($\lambda_1$ to $\lambda_n$) representing a wavelength band having a width of 1 to 20 nm.

6. The method according to one of claims 1 to 5, characterized in that the predetermined wavelength range is at least a part of the region of visible light and/or at least a part of the near-infrared region.

7. An apparatus for the photometric determination of the concentration of one or more substances in a piece of meat comprising:

- means (4, 6, 7) for illuminating a meat surface and for measuring the intensity of light reflected from the meat surface at a plurality of different wavelenghts ($\lambda_1$ to $\lambda_n$) within a predetermined wavelength range,
- calculation means (10) for calculating the concentration of a substance on the basis of the measured intensities of reflected light ($R_1$ to $R_n$), and
- output means (11) for outputting and/or displaying a calculated concentration or corresponding signal,

characterized in that it comprises

- a probe (3) which is determined and shaped to be inserted into a piece of meat whose content is to be measured, said probe being provided with a window (4),
- a first set of optical fibers (7), optically connected at one end to a light source (6), the other end being optically connected to the window (4), for illuminating the meat surface resulting from the insertion of the probe (3) in the meat,
- a second set of optical fibers (8), optically connected at one end to the window (4) for receiving the light reflected from the meat surface, the other end being optically connected to a spectrophotometer (9) selectively registering the light intensities at different wavelengths,
- a computer (10) connected to the output of the spectrophotometer for receiving the registered intensities being measured at different wavelengths ($\lambda_1$ to $\lambda_n$), for insertion of the values in a multi-variable algorithm expressing the concentration of the substance, and for performing the calculation.

8. The apparatus according to claim 7, characterized in that the spectrophotometer (9) includes dispersion means comprising an array of light-sensitive photodetectors for selectively measuring the intensities of reflected light at the wavelengths $\lambda_1$ to $\lambda_n$.

9. The apparatus according to claim 7 or 8, characterized in that the calculation means (10) are adapted to perform the calculation of the respective meat property (C), preferably the concentration of a substance of interest comprised in the meat, in accordance with the following equation:

$$C = k_o + k_1 \cdot R_1 + k_2 \cdot R_2 + k_3 \cdot R_3 + \dots k_n \cdot R_n$$

wherein

- $R_1$, $R_2$, $R_3$, .... $R_n$ are the intensities of reflected light measured at the respective wavelengths $\lambda_1$, $\lambda_2$, $\lambda_3$, .... $\lambda_n$, and
- $k_o$, $k_1$, $k_2$, $k_3$, ....$k_n$ are predetermined constants.

10. The apparatus according to one of claims 7 to 9, characterized in that the calculation means (10) and/or the output means (11) are designed such as to perform a classification of the measured meat pieces on the basis of the calculated values of the meat property (C) and/or to transmit control signals for controlling the transport route of the measured meat pieces and/or to actuate a marking or printing device for marking the meat pieces and/or to display the respective values of the meat property (C).

**Patentansprüche**

1. Verfahren zur photometrischen Bestimmung der Konzentration einer oder mehrerer Substanzen in einem Fleischstück, das folgende Schritte umfaßt:

- Beleuchten einer Fleischoberfläche,

- Messen der Intensität des von der Fleischoberfläche reflektierten Lichts bei einer Vielzahl verschiedener Wellenlängen ($\lambda_1$ bis $\lambda_n$) in einem vorbestimmten Wellenlängenbereich und

- Berechnen der Konzentration einer Substanz auf der Basis der gemessenen Intensitäten des reflektierten Lichts ($R_1$ bis $R_n$) gemäß einem Algorithmus mit mehreren Variablen,

dadurch gekennzeichnet, daß

- eine Sonde in das Fleischstück, dessen Gehalt gemessen werden soll, hineingesteckt wird, wobei die Sonde mit einem Fenster, einem ersten Satz optischer Fasern, mit denen das Fleisch außerhalb des Fensters beleuchtet wird, und einem zweiten Satz optischer Fasern ausgestattet ist, die das von der Fleischoberfläche reflektierte Licht durch das Fenster zu einem die Lichtintensitäten bei verschiedenen Wellenlängen aufzeichnenden Spektralphotometer leiten,

- die Intensitäten des reflektierten Lichts ($R_1$ bis $R_n$) bei verschiedenen Wellenlängen auf einer Fleischoberfläche gemessen werden, die durch das Hineinstecken der Sonde in das Fleisch erzeugt wird,

- die aufgezeichneten Intensitäten ein Berechnungsprogramm in einem Computer übertragen und in einen Algorithmus mit mehreren Variablen eingesetzt werden, der die Konzentration der Substanz angibt, und

- die Konzentration berechnet wird.

2. Verfahren nach Anspruch 1, dadurch gekennzeichnet, daß die Konzentration in Schritt II gemäß der folgenden Gleichung

$$C = k_o + k_1 \cdot R_1 + k_2 \cdot R_2 + k_3 \cdot R_3 + \ldots k_n \cdot R_n$$

berechnet wird, worin bedeuten:

- $R_1$, $R_2$, $R_3 \ldots R_n$ die Intensitäten des bei den einzelnen Wellenlängen $\lambda_1$, $\lambda_2$, $\lambda_3$, .... $\lambda_n$ gemessenen reflektierten Lichts, und
- $k_0$, $k_1$, $k_2$, $k_3 \ldots k_n$ vorbestimmte Konstanten.

3. Verfahren nach einem der Ansprüche 1 und 2, dadurch gekennzeichnet, daß die Intensitäten des reflektierten Lichts ($R_1$ bis $R_n$) bei 30 oder mehr Wellenlängen ($\lambda_1$ bis $\lambda_n$) gemessen werden.

4. Verfahren nach einem der Ansprüche 1 bis 3, dadurch gekennzeichnet, daß die Intensitäten des reflektierten Lichts ($R_1$ bis $R_n$) bei Wellenlängen ($\lambda_1$ bis $\lambda_n$) in einem Wellenlängenbereich von mindestens 100 nm und vorzugsweise mindestens 200 nm gemessen werden.

5. Verfahren nach einem der Ansprüche 1 bis 4, dadurch gekennzeichnet, daß die Intensitäten des reflektierten Lichts ($R_1$ bis $R_n$) bei verschiedenen Wellenlängen ($\lambda_1$ bis $\lambda_n$) gemessen werden, die einen kontinuierlichen Wellenlängenbereich umfassen und die vorzugsweise annähernd gleichmäßig über den vorbestimmten Wellenlängenbereich verteilt sind, wobei jede Wellenlänge ($\lambda_1$ bis $\lambda_n$) vorzugsweise ein Wellenlängenband mit einer Breite von 1 bis 20 nm darstellt.

6. Verfahren nach einem der Ansprüche 1 bis 5, dadurch gekennzeichnet, daß der vorbestimmte Wellenlängenbereich mindestens teilweise im Bereich des sichtbaren Lichts und/oder mindestens teilweise im nahen NIR-Bereich liegt.

7. Vorrichtung zur photometrischen Bestimmung der Konzentration einer oder mehrerer Substanzen in einem Fleischstück, die umfaßt:

- Einrichtungen (4, 6, 7) zur Beleuchtung einer Fleischoberfläche und zur Messung der Intensität des von der Fleischoberfläche bei einer Vielzahl verschiedener Wellenlängen ($\lambda_1$ bis $\lambda_n$) in einem vorbestimmten Wellenlängenbereich reflektierten Lichts,
- Berechnungseinrichtung (10) zur Berechnung der Konzentration einer Substanz auf der Basis der gemessenen Intensitäten des reflektierten Lichts ($R_1$ bis $R_n$) und
- Ausgabeeinrichtung (11) zur Ausgabe und/oder Anzeige einer berechneten Konzentration oder eines entsprechenden Signals,

dadurch gekennzeichnet, daß sie umfaßt:

- eine Sonde (3), die dafür vorgesehen und entsprechend geformt ist, in ein Fleischstück hineingesteckt zu werden, dessen Gehalt gemessen werden soll, wobei die Sonde (3) mit einem Fenster (4) ausgestattet ist,
- einen ersten Satz optischer Fasern (7), die am einen Ende optisch mit einer Lichtquelle (6) und am anderen Ende optisch mit dem Fenster (4) verbunden sind, zur Beleuchtung der Fleischoberfläche, die durch das Hineinstecken der Sonde (3) in das Fleisch erzeugt wird,
- einen zweiten Satz optischer Fasern (8), die am einen Ende optisch mit dem Fenster (4) zur Aufnahme des von der Fleischoberfläche reflektierten Lichts verbunden sind und die am anderen Ende optisch mit einem Spektralphotometer (9) verbunden sind, das selektiv die Lichtintensitäten bei verschiedenen Wellenlängen aufzeichnet,
- einen Computer (10), der mit der Ausgabe des Spektralphotometers verbunden ist, zur Aufnahme der aufgezeichneten Intensitäten, die bei verschiedenen Wellenlängen ($\lambda_1$ bis $\lambda_n$) gemessen wurden, zum Einsetzen der Werte in einen Algorithmus mit mehreren Variablen, der die Konzentration der Substanz angibt, und zur Durchführung der Berechnung.

8. Vorrichtung nach Anspruch 7, dadurch gekennzeichnet, daß das Spektralphotometer (9) ein Dispersionselement einschließt, das eine Anordnung lichtempfindlicher Photodetektoren zur selektiven Messung der Intensität des reflektierten Lichts bei den Wellenlängen $\lambda_1$ bis $\lambda_n$ aufweist.

9. Vorrichtung nach Anspruch 7 oder 8, dadurch gekennzeichnet, daß die Berechnungseinrichtung (10) an die Durchführung der Berechnung der jeweiligen Fleischeigenschaft (C), vorzugsweise der Konzentration einer in dem Fleisch enthaltenen interessierenden Substanz, gemäß der folgenden Gleichung

$$C = k_o + k_1 \cdot R_1 + k_2 \cdot R_2 + k_3 \cdot R_3 + \ldots k_n \cdot R_n$$

angepaßt ist, worin bedeuten:

- $R_1, R_2, R_3, \ldots R_n$ die Intensitäten an reflektiertem Licht, gemessen bei den jeweiligen Wellenlängen $\lambda_1, \lambda_2, \lambda_3, \ldots \lambda_n$, und

- $k_0, k_1, k_2, k_3 \ldots k_n$ vorbestimmte Konstanten.

10. Vorrichtung nach einem der Ansprüche 7 bis 9, dadurch gekennzeichnet, daß die Berechnungseinrichtung (10) und/oder die Ausgabeeinrichtung (11) so konstruiert sind, daß sie eine Klassifizierung der gemessenen Fleischstücke auf der Basis der berechneten Werte der Fleischeigenschaft (C) durchführen und/oder Kontrollsignale zur Kontrolle der Transportwege der gemessenen Fleischstücke übertragen und/oder eine Markierungs- oder Druckvorrichtung zur Markierung der Fleischstücke betätigen und/oder die jeweiligen Werte der Fleischeigenschaft (C) anzeigen.

**Revendications**

1. Procédé de détermination photométrique des concentrations d'une ou de plusieurs substances dans un morceau de viande, consistant à :

- éclairer une surface de la viande,
- mesurer l'intensité de la lumière réfléchie par la surface de la viande pour une pluralité de longueurs d'onde différentes ($\lambda_1$ à $\lambda_n$) dans une gamme prédéterminée de longueurs d'onde, et
- calculer la concentration d'une substance sur la base des intensités mesurées de la lumière réfléchie ($R_1$ à $R_n$) en fonction d'un algorithme à variables multiples,

caractérisé en ce que

- on introduit une sonde dans le morceau de viande dont le contenu doit être mesuré, ladite sonde étant pourvue d'une fenêtre, un premier ensemble de fibres optiques éclairant la viande à l'extérieur de ladite fenêtre, et un second ensemble de fibres optiques guidant la lumière réfléchie par la surface de la viande à travers la fenêtre jusqu'à un spectromètre enregistrant les intensités lumineuses pour différentes longueurs d'onde,
- on mesure, pour différentes longueurs d'onde, sur une surface de la viande, les intensités de

la lumière réfléchie ($R_1$ à $R_n$) résultant de l'insertion de la sonde dans la viande,

- on envoie les intensités enregistrées à un programme de calcul dans un ordinateur et on les introduit dans un algorithme à variables multiples exprimant la concentration de la substance, et
- on calcule la concentration.

2. Procédé selon la revendication 1, caractérisé en ce qu'on calcule la concentration lors de l'étape II conformément à la relation suivante :

$$C = k_o + k_1 . R_1 + k_2 . R_2 + k_3 . R_3 + \ldots k_n . R_n$$

dans laquelle

- $R_1, R_2, R_3, \ldots R_n$ sont les intensités de la lumière réfléchie mesurées pour les longueurs d'onde respectives $\lambda_1, \lambda_2, \lambda_3, \ldots \lambda_n$, et
- $k_o, k_1, k_2, k_3, \ldots k_n$ sont des constantes prédéterminées.

3. Procédé selon l'une des revendications 1 ou 2, caractérisé en ce qu'on mesure les intensités de la lumière réfléchie ($R_1$ à $R_2$) pour 30 longueurs d'onde ou plus ($\lambda_1$ à $\lambda_n$).

4. Procédé selon l'une quelconque des revendications 1 à 3, caractérisé en ce qu'on mesure les intensités de la lumière réfléchie ($R_1$ à $R_n$) pour des longueurs d'onde ($\lambda_1$ à $\lambda_n$) situées dans une gamme d'au moins 100 nm et de préférence d'au moins 200 nm.

5. Procédé selon l'une quelconque des revendications 1 à 4, caractérisé en ce qu'on mesure les intensités de la lumière réfléchie ($R_1$ à $R_n$) pour différentes longueurs d'onde ($\lambda_1$ à $\lambda_n$) qui incluent une gamme continue de longueurs d'onde et sont de préférence réparties de manière approximativement uniforme dans la gamme prédéterminée de longueurs d'onde, chacune des longueurs d'onde ($\lambda_1$ à $\lambda_n$) représentant de préférence une bande de longueurs d'onde possédant une largeur de 1 à 20 nm.

6. Procédé selon l'une quelconque des revendications 1 à 5, caractérisé en ce que la gamme prédéterminée de longueurs d'onde fait au moins partie de la gamme de la lumière visible et/ou fait au moins partie de la zone de la gamme du proche infrarouge.

7. Dispositif pour la détermination photométrique de la concentration d'une ou plusieurs substances dans un morceau de viande, comprenant :

- des moyens (4,6,7) pour éclairer une surface d'une viande et pour mesurer l'intensité de la lumière réfléchie par la surface de la viande pour une pluralité de longueurs d'onde différen-

tes ($\lambda_1$ à $\lambda_n$) dans une gamme prédéterminée de longueurs d'onde,

- des moyens de calcul (10) pour calculer la concentration d'une substance sur la base des intensités mesurées de la lumière réfléchie ($R_1$ à $R_n$), et

- des moyens de sortie (11) pour délivrer et/ou afficher une concentration calculée ou un signal correspondant, caractérisé en ce qu'il comprend

- une sonde (3) qui est déterminée et conformée de manière à être insérée dans un morceau de viande, dont le contenu doit être mesuré, ladite sonde étant pourvue d'une fenêtre (4),

- un premier ensemble de fibres optiques (7), connecté optiquement par une extrémité à une source de lumière (6), l'autre extrémité étant connectée optiquement à la fenêtre (4) pour éclairer la surface de la viande sous l'effet de l'insertion de la sonde (3) dans la viande,

- un second ensemble de fibres optiques (8), connectées optiquement par une extrémité à la fenêtre (4) pour recevoir la lumière réfléchie par la surface de la viande, l'autre extrémité étant connectée optiquement à un spectrophotomètre (9) enregistrant de façon sélective les intensités lumineuses pour différentes longueurs d'onde,

- un ordinateur (10) connecté à la sortie du spectrophotomètre pour recevoir les intensités enregistrées mesurées pour différentes longueurs d'onde ($\lambda_1$ à $\lambda_n$), pour l'insertion des valeurs dans un algorithme à variables multiples exprimant la concentration de la substance, et pour exécuter le calcul.

8. Dispositif selon la revendication 7, caractérisé en ce que le spectrophotomètre (9) comprend des moyens de dispersion comportant un réseau de photodétecteurs photosensibles pour mesurer de façon sélective les intensités de la lumière réfléchie pour les longueurs d'onde ($\lambda_1$ à $\lambda_n$).

9. Dispositif selon la revendication 7 ou 8, caractérisé en ce que les moyens de calcul (10) sont adaptés pour exécuter le calcul de la propriété respective (c) de la viande, de préférence la concentration d'une substance à laquelle on s'intéresse et qui est contenue dans la viande, conformément à la relation suivante :

$$C = k_o + k_1.R_1 + k_2.R_2 + k_3.R_3 + .... k_n.R_n$$

dans laquelle

- $R_1$, $R_2$, $R_3$, ... $R_n$ sont les intensités de la lumière réfléchie mesurées pour les longueurs d'onde respectives $\lambda_1$, $\lambda_2$, $\lambda_3$, ... $\lambda_n$, et

- $k_o$, $k_1$, $k_2$, $k_3$, ... $k_n$ sont des constantes prédé-

terminées.

10. Dispositif selon l'une des revendications 7 à 9, caractérisé en ce que les moyens de calcul (10) et/ou les moyens de sortie (11) sont conçus de manière à exécuter une classification des morceaux de viande mesurés sur la base des valeurs calculées de la propriété (C) de la viande et/ou transmettre des signaux de commande pour commander le trajet d'acheminement des morceaux de viande mesurés et/ou actionner un dispositif de marquage ou d'impression pour marquer les morceaux de viande et/ou afficher les valeurs respectives de la propriété (C) de la viande.

FIG. 1

FIG. 2

FIG. 3

FIG. 4

FIG. 5

EP 0 402 877 B1

EP 0 402 877 B1

FIG. 6

## FIG. 7

## FIG. 8

## FIG. 9